(19) **Europäisches Patentamt**
**European Patent Office**
**Office européen des brevets**

(11) **EP 1 967 524 A1**

(12) **EUROPEAN PATENT APPLICATION**

(43) Date of publication:
**10.09.2008 Bulletin 2008/37**

(51) Int Cl.:
*C07K 5/117* (2006.01) *C07K 7/00* (2006.01)
*C12P 21/06* (2006.01) *A23L 1/305* (2006.01)
*A23J 3/34* (2006.01) *A61K 38/01* (2006.01)
*A61K 38/02* (2006.01) *A61K 38/04* (2006.01)

(21) Application number: **07103545.5**

(22) Date of filing: **06.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Friesland Brands B.V.**
**7943 PE Meppel (NL)**

(72) Inventors:
• **Bonte, Alfred Willy**
**7161 EN Neede (NL)**

• **Geurts, Johannes Marie Wilhelmus**
**7559 NN Hengelo (NL)**

(74) Representative: **van Loon, C.J.J.**
**Vereenigde**
**Johan de Wittlaan 7**
**2517 JR Den Haag (NL)**

<u>Remarks:</u>
The sequence listing, which is published as annex to the application documents, was filed after the date of filing. The applicant has declared that it does not include matter which goes beyond the content of the application as filed.

(54) **Methods for producing ACE-inhibitory peptides from whey and peptides obtained thereby**

(57) The invention relates to methods for providing compounds with an antihypertensive effect. More in particular, the invention relates to releasing ACE (angiotensin I- converting enzyme)-inhibitory peptides from whey proteins. Provided is a method for providing a protein hydrolysate having ACE-inhibitory activity, comprising treating a whey protein-containing substrate with a bacterial heat-labile neutral protease to produce a primary hydrolysate and treating said primary hydrolysate with a thermolysin to produce a secondary hydrolysate. Also provided are hydrolysates and isolated peptides obtainable by said method and uses thereof for the preparation of a medicament for inhibiting ACE activity in a mammal; for lowering the blood pressure; and/or for preventing the occurrence of hypertension.

EP 1 967 524 A1

**Description**

The invention relates to methods for providing compounds having an antihypertensive effect. More in particular, the invention relates to releasing ACE (angiotensin I- converting enzyme)-inhibitory peptides from whey proteins. Further, the invention relates to whey hydrolysates and isolated peptides derived from whey having ACE-inhibitory activity; compositions comprising such whey hydrolysates and/ or peptides; and the use of such hydrolysates and/or peptides, e.g. for the preparation of a medicament for inhibiting ACE activity in a mammal.

Despite major advancements in diagnosis and therapeutics, cardiovascular disease (CVD) remained the leading cause of death in the western industrialized society. Hypertension (high blood pressure) is the most important risk factor for developing a cardiovascular related disease (infarction, stroke, heart failure and/or renal diseases). Effective antihypertensive therapy has made a major contribution to a reduction in the mortality of CVD. Drugs that inhibit or antagonize components of the Renin-Angiotensin-Aldosteron-System (RAAS) had a major effect on reducing the obtained blood pressure in persons who are suffering of hypertension. One of the enzymes active in the RAAS is Angiotensin Converting Enzyme (ACE). This enzyme is a carboxydipeptidase and it catalyzes the conversion of Angiotensin I into the bioactive peptide angiotensin II. Furthermore, the bioactive peptide bradykinin is hydrolyzed into non-functional peptides. The main biological functionality of angiotensin II and bradykinin is related with the regulation of the body blood pressure and the blood flow through important organs like the kidney and the lungs. Important groups of therapeutics that will lower the blood pressure are in fact targeting the ACE enzyme. Pharmaceuticals like for instance captopril and lysinopril inhibit the activity of the ACE enzyme. A common feature of these ACE inhibitors is that the structure of the pharmaceuticals has a great similarity with a dipeptide or a tripeptide. Captopril is a dipeptide (Cys-Pro) and a very potent ACE-inhibitor.
The food industry is looking for new opportunities to develop health promoting food ingredients, so-called functional foods or nutriceuticals. The industry has assigned the reduction of blood pressure in hypertensive consumers as a major area of interest.

It is hypothesized that specific peptides originating from a protein that is available in bulk quantity (casein, whey-, soy and fish protein) can inhibit the ACE enzyme and, as a result, reduce the blood pressure and therefore lower the risk of a CVD related disease. It has been shown that certain protein hydrolysates can inhibit *in vitro* ACE activity. The hydrolysates are obtained by either protease treatment or fermentative activity. There is data available regarding the effect on the *in vivo* blood pressure in rats. This is a commonly used and accepted procedure for measuring blood pressure lowering activity.

Health-promoting food on this subject are now entering the market. For instance, Valio and Calpis bring a product on the market that is obtained by microbial fermentation of milk proteins. Furthermore, DMV has a hydrolysate of casein containing a peptide with a strong ACE inhibiting activity. Most consumer products are commercialized as a milk-based product (milk or yoghurt)

It is not exactly known what structural characteristics ACE-inhibitory peptides should satisfy, but there is some similarity between the peptides that are proven to be ACE inhibitors. These are generally short peptides (2-4 amino acids) with a hydrophobic amino acid on the C-terminal end. Also, the amino acid proline is often mentioned which is then terminally, usually C-terminally present on the peptide.

The Japanese firm Calpis Co. Ltd has obtained ACE-inhibitory peptides through fermentation. These are usually small peptides which are characterized by the presence of the amino acid proline and a high ACE-inhibitory activity. The active peptides obtained by fermentation mainly consist of the sequences which have proline in their structures; namely Val-Pro-Pro or Ile-Pro-Pro (Nakamura, Y. et al. (1996), J. Dairy Sci. 78; 777-783). Other peptides with an antihypertensive activity are described in *inter alia* Nouchikusangiyou, Snow Brand, Symbicom AB and Nisshin Flour Milling Co. Ltd.

It is clear that it is important for a measurable blood pressure decrease that the active peptides / hydrolysates have sufficient ACE-inhibitory activity. The ACE-inhibitory activity of an ACE-inhibitor, such as a peptide or hydrolysate, is usually expressed as an IC50 value. This value indicates the concentration of peptide / hydrolysate necessary to reduce the ACE activity by 50%. The lower the IC50 value of an ACE inhibitor, the stronger the ACE-inhibitory activity.

The object of the invention is to provide hydrolysates having a sufficient ACE-inhibitory activity. More specifically, the invention aims at providing novel methods for the enzymatic production of ACE-inhibitory methods using whey proteins, among others beta-lactoglobulin, as starting material.

The present inventors surprisingly found that the stepwise hydrolysis of a whey protein-containing substrate with two distinct bacterial enzymes (a neutral protease and a thermolysin) can yield a hydrolysate having an IC50 as low as 16 μg/ml. Accordingly, the invention relates to a method for providing a protein hydrolysate having angiotensin-converting enzyme (ACE)-inhibitory activity, comprising:

- treating a whey protein-containing substrate with a bacterial heat-labile neutral protease to produce a primary hydrolysate; and
- treating said primary hydrolysate with a thermolysin to produce a secondary hydrolysate. Thus, provided is a two-step hydrolysis process comprising treatment of a whey protein-containing substrate with a bacterial neutral protease and a thermolysin (EC 3.4.24.27 or EC 3.4.24.4) in a stepwise manner.

It is known that proteinases in various bacterial strains, many of which may be used in the manufacture of fermented dairy products, are capable of releasing ACE-inhibitory peptides from milk proteins (for a review see Meisel and Bockelmann (1999), Ant. van Leeuwen. 76:207-215 or Gobbetti et al. (2000) Appl. Envir. Microbiol. 66: 3898-3904).

For example, WO 03/102195 discloses the use of a combination of one or more endoproteases, in particular a proline-specific endoprotease, with one or more tripeptidases. As an example, a casein hydrolysate is contacted with a serine protease followed by treatment with EndoPro, and finally with the tripeptidase TPAP-A.

WO 02/19837 relates to a process of preparing a whey protein hydrolysate having improved flavour, functionality and ACE-I inhibiting properties. Disclosed is a single step process comprising the treatment of whey protein isolate with one or more heat labile protease(s) until a degree of hydrolysis of no greater than about 10% has been reached, followed by enzyme deactivation. Preferred proteases are Protease 6, Protease A, Protease M, Peptidase, Neutrase, Validase and AFP 2000.

JP 06128287 discloses the preparation of opioid peptides with ACE inhibiting activity by hydrolyzing a milk protein (e.g. cow's milk beta-casein) with a neutral or an alkaline protease derived from a microorganism and then recovering a fraction having the opioid activity.

US 6,998,259 discloses a process for preparing an angiotensin-converting enzyme (ACE)-inhibiting composition comprising:

(a) preparing an aqueous solution of a whey protein fraction produced by ion exchange and trypsin; (b) holding said solution under conditions effective for partially hydrolyzing said whey protein fraction to provide a hydrolysate having increased ACE-inhibiting activity; (c) stopping the hydrolyzation when a degree of hydrolysis is reached within the range of from 5.5 to 6.5%, wherein said hydrolysate comprises a mixture of peptides having a specific Molecular Weight Profile, as determined by HPLC. Enzymes used in US 6,998,259 include Trypsin VI, P Amano 6 and Multifect, having very different specificities. Trypsin is known to cleave only the peptidic bonds in the vicinity of Arg and Lys, whereas the two other enzymes have a much broader specificity and will lead to a greater number of shorter peptides.

WO2006/025731 provides a method for enzymatically producing a protein hydrolysate with ACE-inhibitory activity, characterized by treatment of a beta-lactoglobulin (BLG) -containing substrate with a broad-spectrum endoprotease in a first reaction step followed by treatment with a proline-specific endoprotease in second reaction step.

WO2006/005757 relates to a process to produce a composition which comprises the tripeptide IPP from a protein whereby the weight ratio of IPP to VPP produced from the protein is at least 5: 1 , preferably at least 10:1 and more preferably at least 20:1, which comprises the use of an enzyme activity which has proline specific endoprotease activity or prolyl oligopeptidase activity and an enzyme activity which is capable of hydrolysing the bond at the amino terminal side of a -I-P-P- sequence. Milk protein, preferably casein, can be used as starting material.

WO04/098310 describes a process for the preparation of a hydrolysed casein product comprising tripeptides VPP, IPP and and/or LPP, wherein a substrate comprising casein or casein fragments is subjected to an enzyme derived from *Aspergillus.* Preferably, the enzyme has a X-Prolyl dipeptidyl aminopeptidase activity or 400 U/kg or more.

Thus, although the single step or multistep hydrolysis of a milk protein using one or more different enzymes is known, the treatment of a whey protein with the specific enzyme combination as disclosed herein for releasing ACE-inhibitory peptides has not been disclosed or suggested before.

Heat-labile neutral bacterial proteases are known in the art. By heat - labile is meant that the enzyme is susceptible to irreversible deactivation at relatively moderate temperatures as would be appreciated by a person skilled in the art. An enzyme having a substrate cleavage specificity defined as P1= Leu, Val or Phe residue may be used, where P1 is the residue on the N-terminal side of the scissile bond. Suitable heat labile bacterial neutral proteases include those derived from a *Bacillus* spp., in particular *Bacillus subtilis* or *Bacillus amyloliquefaciens.* In a specific aspect, a method of the invention comprises the use of a neutral protease which is marketed by NovoZymes under the tradename Neutrase(R), in particular Neutrase 0.5L, or an enzyme with similar properties.

Thermolysin (EC 3.4.24.27) is a heat-stable metalloproteinase obtained from the bacterium *Bacillus thermoproteolyticus,* which hydrolyses the N-terminal amide bonds of hydrophobic amino acid residues in proteins, such as Leu, Ile, Val and Phe (P1'= Ile, Leu, Val or Phe). It has a relatively high optimum temperature, ranging from about 65-75°C. In a specific aspect, the present invention is practiced using thermolysin produced by *B. stearothermophilus (B. thermoproteolyticus* variant Rokko) or a thermolysin with similar properties.

The person skilled in the art will be able to choose the conditions that are suitable for carrying out the enzyme treatments. Substrate hydrolysis with heat-labile neutral protease is typically carried out at a pH of between about 3.5 and about 9.0, preferably at about pH 6.0 to 8.0. Suitable incubation temperatures range from about 30-65°C, preferably from about 50-60°C.

Incubation with thermolysin can be performed at temperatures up to about 80°C, although the optimum temperature is 65-70°C. The pH can range from about 5 to about 9, the optimum pH being 7.0-8.5. Thermolysin is protected with calcium ions and inhibited by chelating agents like EDTA.

One or more enzymes used in a method of the invention can be immobilised on an inert support, for example on chitosan particles, carrageenan particles, Eupergit or any other suitable inert support material. The enzyme(s) may be attached to the support material by cross-linking.

Treatment of a whey protein-containing substrate with a heat-labile neutral protease results in a first hydrolysate, which preferably has a relatively low degree of hydrolysis (DH), i.e. the percentage of peptide bonds cleaved by enzymic action is preferably less than about 10%. The DH of a hydrolysate can be determined by manners known in the art. In a preferred aspect, treatment with neutral protease is conducted, for example using Neutrase, to yield a hydrolysate with a DH between about 3 and 7%, such as 3-5% as determined by the method of Adler-Nissen (Enzymic Hydrolysis of Food Proteins. ISBN 0-85334-386-1, pg. 115-124). Said method uses the following equation: $DH = B \times N_b \times 1/\alpha \times 1/MP \times 1/H_{tot} \times 100\%$
wherein

B = sodium hydroxide consumption (ml)
Nb = Normality of the sodium hydroxide solution
$\alpha$ = Average degree of dissociation of $\alpha$-NH-groups
$H_{tot}$ = Total number of peptide bonds in the protein substrate (meq/g protein)
MP = mass of protein in hydrolysis (g)

The enzyme used in the first reaction step is typically inactivated prior to subjecting the first hydrolysate to the second enzyme, among others to avoid hydrolysis of the second enzyme by the first enzyme. After completion of the second hydrolysis, the second enzyme (thermoase) may be inactivated. Inactivation may comprise reversible or irreversible inactivation. Enzyme inactivation is readily achieved by denaturation of the enzyme, typically by brief exposure to a high temperature. Heat inactivation for instance comprises heating the first hydrolysate comprising the first enzyme for several seconds to minutes at a temperature of about 95-130°C. For example, a so-called UHT treatment of heating approximately 10 seconds at 121C° is used. This also allows for microbial control. Of course, heat inactivation of heat-labile neutral protease requires lower temperatures as compared to heat inactivation of thermolysin. A suitable temperature range which could be used for inactivation of the first enzyme is a heat labile bacterial neutral protease is in the order of about 55-70°C, preferably about 65°C.

Alternatively, enzyme may be inactivated by simply removing it from the reaction mixture. For example, when the enzyme is immobilised on an inert support it may be subsequently separated out of the first hydrolysate, e.g, by membrane filtration, in order to achieve loss of first enzyme activity.

Also, first enzyme may be separated out of the first hydrolysate with the use of ultrafiltration (UF) technology, preferably using an ultrafiltration membrane with a nominal molecular weight cut-off in the range of about 10-500 kDa, preferably about 10-200 kDa, once hydrolysis has reached the desired level.

Any type of whey protein-containing substrate can be used as starting material. These include preparations referred to as "whey protein isolate" (WPI) or "whey protein concentrate" (WPC). WPC's are typically obtained by ultrafiltration of concentrated whey. Whey constitutes 80 to 90% of the total volume of milk entering the production process and contains about 50% of the nutrients of the original milk such as proteins, lactose, vitamins and minerals. In the past, whey has always been considered waste of the cheese industry, but the discharge of cheese whey causes large environmental problems. However, because the proteins have good functional properties, nowadays the interest in the use of whey for the extraction thereof is great.

Generally speaking only 0.55% of the whey consists of proteins. On a dry matter basis, this is approximately 10%. The whey proteins are a heterogeneous mixture, of which the main proteins are β-lactoglobulin (BLG; about 55%), α-lactoglobulin (ALA; about 16%), blood serum albumin (BSA; about 5%) and immunoglobulins (Ig; about 12%). In order to be able to increase the dry matter content of whey to at least approximately 50-60%, whey is concentrated or dried. A whey protein concentrate (WPC) is made by fractionating and concentrating whey by means of ultrafiltration. As further concentrating takes place, different levels of protein arise in the whey protein concentrate. Industrially produced WPCs are classified into the categories low-protein WPC (protein content between 25-40%), medium-protein WPC (protein content between 45-60%) and high-protein WPC (protein content between 60-80%).

Besides normal WPC comprising the complete spectrum of whey proteins, a WPC can be used that is enriched for one or more specific whey proteins, for instance β-lactoglobulin (BLG)-enriched WPC or α-lactoglobulin (ALA)-enriched WPC. In one embodiment, a method of the invention comprises the stepwise enzymic treatment of a WPC enriched in ALA. This product is obtained after specific precipitation of ALA from a normal WPC. After centrifugation and ultrafiltration a WPC is obtained with a relatively high level of ALA. An example of ALA-enriched WPC is the product sold under the trade name Vivinal Alpha™.

A whey protein concentrate enriched with beta-lactoglobulin (BLG) is an attractive starting material in a method according to the invention. This product is obtained as "by-product" during the manufacture of ALA-enriched WPC as described above. Preferably, a method according to the invention is used on a substrate consisting of a mixture of proteins of which at least 25% by weight based on total protein is BLG, such as 30% or 35% BLG, preferably at least 40% BLG, such as 45%, 50% or 55%, more preferably at least 70% BLG, such as 75% or 90%. Also, (purified) BLG can be used as a substrate. However, it goes without saying that the production costs of an ACE-inhibitory hydrolysate will be higher as a more purified protein substrate is used as a starting material.

Suitable commercial WPCs for use as starting material include Hiprotal 875; Hiprotal 880, Hiprotal 535 and Hiprotal 580, all available from Friesland Foods Domo, Zwolle, the Netherlands.

The protein concentration at which the whey protein containing substrate is incubated with first or second enzyme can vary, depending among others on the type and amount of enzyme used. The protein concentration can be the same or different for the first and second incubation mixture. For example, a first hydrolysate is prepared at a protein concentration of from about 1 to about 50 mg per 100 ml, preferably from about 5 to about 15 mg/ml, such as 10 mg/ml. The obtained first hydrolysate may be treated with second enzyme as such (i.e. at the same protein concentration) or it may subsequently be diluted, for example 2- to 10-fold, prior to contacting it with the second enzyme, using water or any other suitable aqueous liquid.

In a specific aspect, a first hydrolysate is prepared by treating a solution comprising whey protein at about 10 g/100 ml using Neutrase, followed by dilution to about 3-4 g/100 ml prior to a treatment with thermolysin.

In a further aspect, the invention provides a protein hydrolysate having angiotensin-converting enzyme (ACE)-inhibitory activity, obtainable with a method as described hereinabove. This hydrolysate is characterized by a strong ACE-inhibitory activity (see Tables 1 and 2 below) and can therefore be used with advantage for inhibiting ACE activity in mammals, such as humans. A hydrolysate is also suitable for veterinary application, for instance in a cat or dog. Since inhibition of ACE has an antihypertensive effect in vivo, an ACE-inhibitory hydrolysate can be used for the treatment of hypertension.

A hydrolysate according to the invention can be processed into different products, such as liquid and dry products. If desired, downstream processing takes place, for instance in the form of an ultrafiltration (UF) treatment. The enzymes have a much larger molecular weight than the peptides and can be separated from the ACE-inhibitory peptides by means of UF. An additional advantage of a UF step is that the protein substrates which have not been degraded by the enzymes can also be separated from the biologically active hydrolysate.

Also provided is a whey-derived peptide having angiotensin-converting enzyme (ACE)-inhibitory activity, obtainable by subjecting a whey hydrolysate according to the invention to a purification procedure wherein individual peptides (whey protein fragments) are isolated. Suitable methods are known in the art and typically include one or more steps of reversed phase (RP-) HPLC. The structure of isolated peptide(s) can be elucidated using various analytical techniques, for example NMR or mass spectrometry (MS). The invention therefore also relates to a peptide having ACE-inhibitory activity obtainable by a method comprising treating a whey protein-containing substrate with a bacterial heat-labile neutral protease to produce a primary hydrolysate, treating said primary hydrolysate with a thermolysin to produce a secondary hydrolysate as described herein above, and further comprising the steps of fractionating the secondary lysate into individual fractions comprising one or more peptides and identifying the amino acid sequence of at least one peptide having ACE-inhibitory activity.

As is exemplified in the Examples below, several ACE-inhibitory peptide fractions can be obtained according to the stepwise enzyme treatment according to the invention. In one embodiment, a fraction comprising one or more whey-derived peptides is provided which can inhibit 30% or more in an *in vitro* ACE assay. Preferably, a fraction shows at least 40%, more preferably at least 50%, most preferably at least 60% inhibition.

Analysis of the peptide(s) present in some of the inhibitory fractions revealed the identity of the at least the following amino acid sequences (all indicated by the standard one-letter code): VAGTWYS, VAGTWYSL, LDIQK, WYSL, IAEKTKIPAV, AASDISL, VAGTW, ASDISL, VYVEE, LKALPM, LIVTQT, IIVTQT, DAQSAPL, VLDTDY, LVLDTDYKKY, DTDYKKY and LKPTPEG.

The invention therefore provides a purified, synthetic or isolated peptide selected from the group consisting of VAGTWYS, VAGTWYSL, LDIQK, WYSL, IAEKTKIPAV, AASDISL, VAGTW, ASDISL, VYVEE, LKALPM, LIVTQT, IIVTQT, DAQSAPL, VLDTDY, LVLDTDYKKY, DTDYKKY and LKPTPEG, and functional peptide analogs thereof which show in vitro ACE-inhibitory activity.

In a preferred embodiment, a peptide selected from the group consisting of VAGTWYS, VAGTWYSL, WYSL, IAEKTKIPAV, AASDISL, VAGTW, ASDISL, VYVEE, LKALPM, LIVTQT, IIVTQT, DAQSAPL, VLDTDY, LVLDTDYKKY, DTDYKKY, LKPTPEG and functional peptide analogs thereof which show *in vitro* ACE-inhibitory activity is provided. In a specific aspect, said peptide is selected from the group consisting of WYSL, IAEKTKIPAV, AASDISL, ASDISL, VYVEE, LKALPM, LIVTQT, IIVTQT, DAQSAPL, LKPTPEG, and functional peptide analogs thereof which show in vitro ACE-inhibitory activity.

As used herein, a "purified, synthetic or isolated" peptide is one that has been purified from a natural or biotechnological source, or, more preferably, is synthesized as described herein.

As used herein, a "functional analogue" of a peptide preferably has the same size as the peptide from which it is derived and thus rather made by amino acid substitutions and/or modifications than by deletions or addition of amino acids. Also, as used herein, a "functional analogue" of a peptide, does not refer to a larger protein or peptide merely containing an amino acid sequence identified as an ACE-inhibitory peptide that is flanked by more amino acids at one or both sides.

For instance, a peptide analog could, in one embodiment be: NT - VAGTWYS-CT wherein NT at the N-terminus is selected from the group of H--, CH3--, an acyl group, or a general protective group; and / or CT at the C-terminus is selected from the group of -OH, --$OR^1$, --$NH_2$, --$NHR^1$, --$NR^1 R^2$, or --$N(CH_2)_{1-6} NR^1 R^2$, wherein $R^1$ and $R^2$, when present, are independently selected from H, alkyl, aryl, (ar)alkyl, and wherein $R^1$ and $R^2$ can be cyclically bonded to one another.

Peptide analogs also encompass equivalent compounds having the same or equivalent side chains as the particular amino acids sequence disclosed above, and arranged sequentially in the same order as the peptides, but joined together by non-peptide bonds, e.g., by isosteric linkages such as the keto isostere, hydroxy isostere, diketo isostere, or the keto-difluoromethylene isostere.

Also provided are peptides in the form of an acceptable salt. As used herein, "acceptable salt" refers to salts that retain the desired activity of the peptide or equivalent compound, but preferably do not detrimentally affect the activity of the peptide or other component of a system in which uses the peptide. Examples of such salts are acid addition salts formed with inorganic acids, for example, hydrochloric acid, hydrobromic acid, sulfuric acid, phosphoric acid, nitric acid, and the like. Salts may also be formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, and the like. Salts may be formed with polyvalent metal cations such as zinc, calcium, bismuth, barium, magnesium, aluminum, copper, cobalt, nickel and the like or with an organic cation formed from N,N'-dibenzylethylenediamine or ethylenediamine, or combinations thereof (e.g., a zinc tannate salt).

The peptide or analogs according to the invention may be prepared in a manner conventional for such compounds. To that end, suitably N alpha protected (and side-chain protected if reactive side-chains are present) amino acid analogs or peptides are activated and coupled to suitably carboxyl protected amino acid or peptide derivatives either in solution or on a solid support. Protection of the alpha-amino functions generally takes place by urethane functions such as the acid-labile tertiary-butyloxycarbonyl group ("Boc"), benzyloxycarbonyl ("Z") group and substituted analogs or the base-labile 9-fluoremyl-methyloxycarbonyl ("Fmoc") group. The Z group can also be removed by catalytic hydrogenation. Other suitable protecting groups include the Nps, Bmv, Bpoc, Aloc, MSC, etc. A good overview of amino protecting groups is given in The peptides, Analysis, Synthesis, Biology, Vol. 3, E. Gross and J. Meienhofer, eds. (Academic Press, New York, 1981). Protection of carboxyl groups can take place by ester formation, for example, base-labile esters like methyl or ethyl, acid labile esters like tert. butyl or, substituted, benzyl esters or hydrogenolytically. Protection of side-chain functions like those of lysine and glutamic or aspartic acid can take place using the aforementioned groups. Protection of thiol, and although not always required, of guanidino, alcohol and imidazole groups can take place using a variety of reagents such as those described in *The Peptides, Analysis, Synthesis, Biology, id.* or in Pure and Applied Chemistry, 59(3), 331-344 (1987). Activation of the carboxyl group of the suitably protected amino acids or peptides can take place by the azide, mixed anhydride, active ester, or carbodiimide method especially with the addition of catalytic and racemization-suppressing compounds like 1-N-N-hydroxybenzotriazole, N-hydroxysuccinimide, 3-hydroxy-4-oxo-3,4-dihydro-1,2,3,- benzotriazine, N-hydroxy-5 norbornene-2,3-dicar-boxyimide. Also, the anhydrides of phosphorus based acids can be used. *See, e.g., The Peptides, Analysis, Synthesis, Biology, supra* and Pure and Applied Chemistry, 59(3), 331-344 (1987). It is also possible to prepare the compounds by the solid phase method of Merrifield. Different solid supports and different strategies are known *see, e.g.* Barany and Merrifield in The Peptides, Analysis, Synthesis, Biology, Vol. 2, E. Gross and J. Meienhofer, eds. (Acad. Press, New York, 1980); Kneib-Cordonier and Mullen, Int. J. Peptide Protein Res., 30, 705-739 (1987); and Fields and Noble, Int. J. Peptide Protein Res., 35, 161-214 (1990). The synthesis of compounds in which a peptide bond is replaced by an isostere, can, in general, be performed using the previously described protecting groups and activation procedures. Procedures to synthesize the modified isosteres are described in the literature, e.g., for the --$CH_2$--NH-- isostere and for the --CO--$CH_2$ -- isostere.

Removal of the protecting groups, and, in the case of solid phase peptide synthesis, the cleavage from the solid support, can take place in different ways, depending on the nature of those protecting groups and the type of linker to the solid support. Usually deprotection takes place under acidic conditions and in the presence of scavengers. *See, e.g.* volumes 3, 5 and 9 of the series on *The Peptides Analysis, Synthesis, Biology, supra.*

Another possibility is the application of enzymes in synthesis of such compounds; for reviews see, *e.g.,* H.D. Jakubke in The Peptides, Analysis, Synthesis, Biology, Vol. 9, S. Udenfriend and J. Meienhofer, eds. (Acad. Press, New York, 1987).

Although possibly not desirable from an economic point of view, ACE-inhibitory peptides according to the invention could also be made according to recombinant DNA methods. Such methods involve the preparation of the desired peptide thereof by means of expressing recombinant polynucleotide sequence that codes for one or more of the peptides in question in a suitable microorganism as host. Generally the process involves introducing into a cloning vehicle (e.g., a plasmid, phage DNA, or other DNA sequence able to replicate in a host cell) a DNA sequence coding for the particular peptide(s), introducing the cloning vehicle into a suitable eukaryotic or prokaryotic host cell, and culturing the host cell thus transformed. When a eukaryotic host cell is used, the compound may include a glycoprotein portion.

As used herein, a "functional analogue" of a peptide includes an amino acid sequence, or other sequence monomers, which has been altered such that the functional properties of the sequence are essentially the same in kind, not necessarily in amount. An analog can be, for instance, generated by substitution of an L-amino acid residue with a D-amino acid residue. This substitution, leading to a peptide that does not naturally occur in nature, can improve a property of an amino acid sequence. It is, for example, useful to provide a peptide sequence of known activity of all D-amino acids in retro inversion format, thereby allowing for retained activity and increased half-life values. By generating many positional variants of an original amino acid sequence and screening for a specific activity, improved peptide derivatives comprising such D-amino acids can be designed with further improved characteristics. It has been shown in the art that peptides that are protected by D-amino acids at either one or both termini were found to be more stable than those consisting of L-amino acids only. Other types of modifications include those known in the art of peptide drug development to have beneficial effects for use of the peptide in a nutritional of pharmaceutical composition. These effects may include improved efficacy, altered pharmacokinetics, increasing stability resulting in a longer shelf-life and less stringent cold chain handling requirements.

The functionality of a peptide or a functional analogue thereof can be determined using an in vivo and/or in vitro testing. In vitro testing of ACE-inhibitory activity is preferred. In one embodiment, a candidate peptide analog is subjected to comparative testing using a reference or control peptide, for instance a peptide analog consisting solely of L-amino acids. A suitable test comprises determining the capability of the candidate peptide to inhibit hydrolysis of Hippuryl-His-Leu by commercially available ACE.

It may be assumed that, after oral intake, the peptide(s) present in a hydrolysate according to the invention end up in the intestinal tract in intact or virtually intact form, to subsequently be taken up into the bloodstream. Optionally, a hydrolysate or peptide according to the invention can be used in combination with one or more other ACE inhibitors. In a further aspect of the invention, the ACE-inhibitory hydrolysate or isolated peptide(s) is preventively used to prevent hypertension, for instance in a person with a genetic disposition for hypertension or if medicines are used which cause hypertension as a side effect.

In addition to being effective with hypertension, ACE inhibitors also prove to be effective with heart failure. An ACE-inhibitory hydrolysate obtainable by a method according to the invention, or an isolated peptide (analog) can therefore also be used with heart problems, for instance after a coronary to prevent recurrence.

The invention also provides a composition comprising an ACE-inhibitory hydrolysate and/or peptide according to the invention and a suitable carrier, optionally together with one or more additional beneficial nutritional or therapeutical ingredients. Such a composition with an ACE-inhibitory / antihypertensive activity is, for instance, a pharmaceutical composition or a food or luxury food. An ACE-inhibitory composition may have a solid or a liquid form. It is, for instance, a powder or a tablet. Preferably, the ACE-inhibitory composition according to the invention has a liquid form, such as a liquid milk product, a fruit juice or another type of drinkable product which can *inter alia* be used to prevent or treat hypertension. Thus, the invention also provides a treatment method for inhibiting ACE activity in a mammal, comprising the, preferably oral, administration of an ACE-inhibitory hydrolysate obtainable by a method of the invention, a peptide, peptide analog, or a composition comprising either one of them in any combination, in an amount and with a frequency which is sufficient to inhibit the ACE activity or at least alleviating one or more symptoms associated with increased ACE-activity.

Still another aspect of the present invention relates to a treatment method to reduce symptoms of hypertension in a mammal, comprising the, preferably oral, administration of a hydrolysate or composition according to the invention in an amount and with a frequency which is sufficient to inhibit the symptoms of hypertension.

Finally, the invention relates to the use of a hydrolysate and/or peptide (analog) according to the invention for the preparation of a medicament for (prophylactically) treating or preventing heart and vascular diseases. In particular, the medicine to be prepared is a medicine for inhibiting ACE activity in a mammal (e.g. human or pet); a medicine for lowering the blood pressure; and/or a (prophylactic) medicine for the occurrence of hypertension.

LEGENDS TO THE FIGURES

Figure 1: Typical hydrolysis pattern of Hiprotal 535 with Neutrase. Protein concentration was 100 mg/ml.

Figure 2: Typical hydrolysis pattern of a primary hydrolysate with Thermoase; Peptide concentration= 40 mg/ml and E/S= 0,01.

EXPERIMENTAL PART

**Example 1: _In vitro_ measurement of ACE inhibition**

The in vitro measurement of the ACE inhibitory activity of hydrolysates was carried out as follows:

- The substrate used for the measurement of ACE activity is Hippuryl-His-Leu from Sigma (H-1635)
- This product (approx. 40 mg) is dissolved in 14 ml of assay buffer consisting of 0.1M borate, 0.3 M sodium chloride at pH 8.3.
- The final assay mixture consists of 200 $\mu$l substrate solution and 800 $\mu$l assay buffer.

The assay is carried out in a Carry 100 (Varian) spectrophotometer at 37°C. At the start of the incubation, 20 $\mu$l ACE (1U/ml; Sigma) is added. ACE enzyme activity is monitored at wavelength of 260 nm. The obtained ACE activity was calculated between 2 and 11 minutes of incubation. The ACE activity is defined as the increase of E260nm per minute incubation.
For the measurement of the inhibitory effect of whey hydrolysate, part of the buffer volume is replaced by a solution of the hydrolysate.
In the presence of hydrolysate to be tested, the inhibition of this activity is measured and expressed as the required amount of product needed to reduce the activity of the enzyme by 50%. This inhibition is determined for four different concentrations of the product to be measured.

**Example 2: Whey protein containing substrate**

Unless indicated otherwise, the whey protein-containing substrate Hiprotal 535 (Friesland Foods Domo) was used as starting material. This is a whey protein concentrate rich in beta-lactoglobulin. It contains 56% lactose, 35% protein, 3% minerals, 2% organic milk salts, 0.5% fat and 3.5% moisture.
Characterization of the proteins by RP-HPLC revealed the following composition:

| | | |
|---|---|---|
| $\alpha$-Lactalbumine | (RP-HPLC) | <0.01% |
| $\beta$-Lactoglobuline | (RP-HPLC) | 27,21% |
| CaseinoMacropeptide | (RP-HPLC) | 5.95% (primarily CMP-A) |
| Protein | (Lowry) | 32.7% |

**Example 3: Preparation of primary hydrolysate**

For the liberation of ACE-inhibiting peptides from Hiprotal 535 a primary hydrolysate was prepared upon incubation with Neutrase 0.5L purchased from Novozymes. The optimal conditions for Neutrase incubation are 50°C and pH=7.0. The objective of the first experiment was to determine the dynamics of a neutrase-catalyzed hydrolysis. In this experiment 10 g protein (28,6g product) was dissolved in a final volume of 100 ml water. The complete solution was placed in the titration stand of the pH-Stat. The starting pH of the solution was approximately pH= 6.45. After equilibration to a temperature of 50°C, a pretitration was carried out with the pH-Stat until the desired pH = 7.0 was reached. At that point 100$\mu$l Neutrase was added to the solution and the incubation was started. The titrator continuously monitored the pH of the reaction. The pH is maintained at the preset value by the controlled addition of sodiumhydroxyde. The degree of hydrolysis (DH) of the proteinhydrolysate can be directly calculated from the base consumption (Adler-Nissen, Jens; Enzymic hydrolysis of food proteins; ISBN 0-85334-386-1; blz 115-124).
The equation that is used:

$$DH = B \times N_b \times 1/\alpha \times 1/MP \times 1/H_{tot} \times 100\%$$

Where:

B = sodium hydroxide consumption (ml)
Nb = Normality of the sodium hydroxide solution

$\alpha$ = Average degree of dissociation of $\alpha$-NH-groups
$H_{tot}$ = Total number of peptide bonds in the protein substrate (meq/g protein)
MP = mass of protein in hydrolysis (g)

For the determination of DH, a value of 8,8 was used for MP. The value of $1/\alpha$ was dependent of the applied temperature and the actual pH during the incubation. For pH = 8, 50°C $1/\alpha$ was 1,13 and for pH=7, 50°C $1/\alpha$ was 2,27

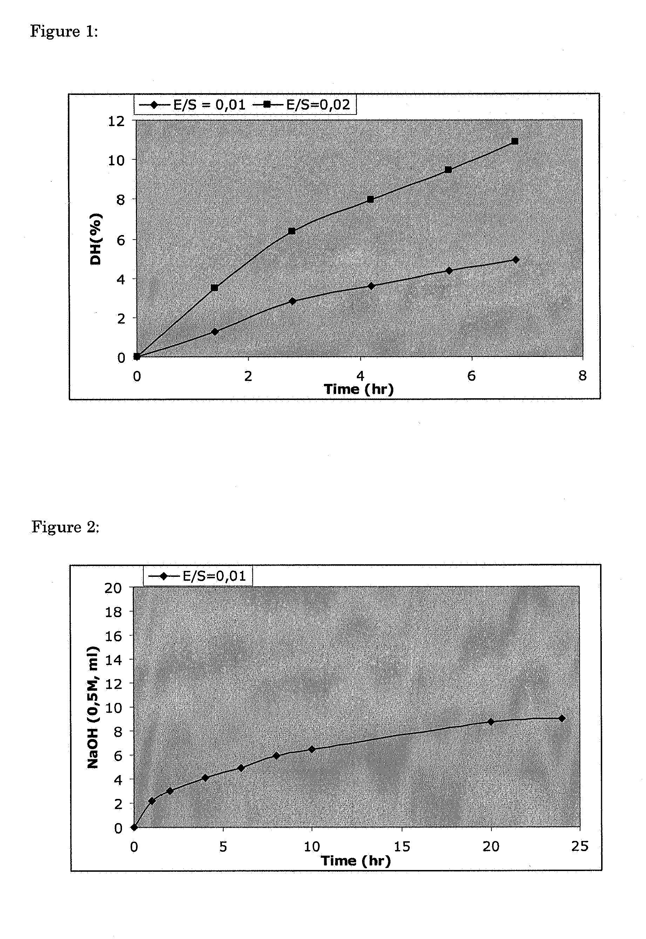

A typical hydrolysis-pattern of Hiprotal 535 with Neutrase is presented in the Figure 1. In the first few minutes of the incubation, hydrolysis is running at a relatively high speed. At a certain moment, velocity is slowing down considerably. When approximately 5% of the available peptide bonds were hydrolyzed, the obtained enzyme activity increases again. Without wishing to be bound by theory, it is believed that during the beginning of the incubation, the conformation of the substrate is inhibiting the enzyme. At a certain moment (DH=5%) the conformation of the substrate changes and makes it more vulnerable for proteolytic attack.

**Experimental data degree of hydrolysis as a function of time:**

| | For Neutrase incubation (E/S= 1:100): | For E/S= 1:50 |
| --- | --- | --- |
| | %DH | %DH |
| t=0 h | 0 | 0 |
| t=1,4h | 1,29 | 3,51 |
| t=2,8h | 2,84 | 6,34 |
| t=4,2h | 3,61 | 7,98 |
| t=5,6h | 4,39 | 9,45 |
| t=6,8h | 4,90 | 10,91 |
| t=17h | 18,3 | |

To determine the effect of the extend of hydrolysis in the primary hydrolysate, three different samples were prepared each with a different degree of hydrolysis (A=5.0%, B=10.6% and C=18.3%). RP-HPLC of the obtained hydrolysates showed that the degradation of protein by the enzyme neutrase was running at a relatively slow yet controllable reaction rate.

**Example 4: Preparation of a secondary hydrolysate**

The obtained primary hydrolysate had a peptide/protein concentration of 10g/100ml. Prior to incubation with the second enzyme thermolysin, the primary hydrolysate was diluted with water to a final concentration of 10 g protein/250ml. The thermolysin used was Thermoase PC10F purchased from Amano.

The conditions used for Thermoase PC10F incubation were pH=8.0 and 50°C. To start the secondary hydrolysis, 100 ml of primary hydrolysate was placed in the pH-Stat instrument. With a pre-titration the pH of the solution was adjusted to pH=8.0. The incubation was started by the addition of a known amount of thermolysin enzyme. Typically, an enzyme to substrate (E/S) ratio of 1/100 or 1/50 was used. A typical hydrolysis pattern of a primary hydrolysate with the enzyme Thermoase is shown in Figure 2, showing that hydrolysis of the primary hydrolysate with Thermoase was running at a relatively high speed. Furthermore it can be concluded that after prolonged incubation with Thermoase a high degree of hydrolysis could be obtained.

Several secondary hydrolysates were prepared, each with a different degree of hydrolysis. To that end, aliquots were taken from the secondary hydrolysate after 60 , 120 and 180 minutes of incubation with thermoase.

The samples were diluted with water to a final protein/peptide concentration of 10mg/ml. The enzyme was inactivated by placing the diluted hydrolysate for 5 minutes in a waterbath (95°C).

As a control, a secondary hydrolysate was also prepared with Protease A as second enzyme the enzyme. This enzyme has a specificity for Ile, Leu or Val at the P1-position. Samples were stored in the refrigerator until the determination of ACE-inhibiting activity. The ACE inhibiting activity was analyzed with the Hip-His-Leu assay described above. Table 1 shows the percentage ACE-inhibition obtained at an inhibitor (peptide) concentration of the hydrolysate of 20 $\mu$g/ml.

Table 1: ACE inhibition by secondary whey protein hydrolysates.

| Primary Hydrolysate | Secondary Hydrolysate | % ACE inhibition (I=20μg/ml) | | |
|---|---|---|---|---|
| | | t=60' | t=120' | t=180' |
| A Neutrase DH=5.0% | Thermoase (1/100) | 31 | 46 | 54 |
| B Neutrase DH=10,6% | Thermoase (1/100) | 30 | 38 | 40 |
| B Neutrase DH=10,6% | Protease A (1/100) | 17 | 25 | 20 |
| C Neutrase DH=18,3% | Thermoase (1/100) | 17 | 26 | 32 |
| C Neutrase DH=18,3% | Protease A (1/100) | 24 | 33 | 29 |
| D Alcalase DH=12,2% | Thermoase (1/100 | 37 | 32 | 30 |

Table 1 shows that incubation of primary hydrolysate with Thermoase results in the release of ACE inhibiting activity which increases up to at least 3 hours of incubation. The best effect was seen with the primary hydrolysate A (Neutrase DH=5.0%).
In contrast, treatment of the same primary hydrolysates with the fungal Protease A (metal proteinase; EC 3.4.24.39) instead of thermolysin to produce a secondary hydrolysate resulted in hardly any improvement with respect to the release of ACE inhibiting peptides.
As a further comparative example, a primary hydrolysate was prepared with Alcalase, a broad range proteolytic enzyme. After treatment of this primary hydrolysate with thermoase, hardly any improvement was seen on obtained ACE inhibiting activity (Table 1, sample D).

Based on the results of Table 1 it was hypothesized that the generation of ACE inhibiting peptides would continue even further upon prolonged incubation with Thermoase. To test this hypothesis, an additional hydrolysate was prepared with an incubation time of 23 hours. ACE inhibiting activity in this hydrolysate was even stronger.

In the next experiment, the IC50 values from a number of secondary hydrolysates were determined. A primary hydrolysate obtained by treating BLG-enriched whey protein with using Neutrase (final DH 5.0 or 10.6%) was subsequently contacted for the indicated time periods (1, 3 or 23 hours) with thermoase at the indicated E/S ratios. IC50 values were determined in vitro using the assay described above. Results are shown in Table 2. Further testing revealed that virtually no changes were observed between 12 and 23 hours of incubation, and that an IC50 of approximately 23 μg/ml was obtained after a 12 hour treatment with thermoase.

Table 2: ACE-inhibitory activity (expressed as IC50) of hydrolysates prepared using the enzyme combination of the invention.

| Primary Hydr. | | Secondary Hydr. | | Ic50(μg/ml) |
|---|---|---|---|---|
| A | Neutrase DH 3.0% | Thermoase (1/100) | 1hr | 42 |
| A | | Thermoase (1/100) | 3hr | 31 |
| A | | Thermoase (1/50) | 23hr | 23 |
| B | Neutrase DH=10.6% | Thermoase (1/100) | 3hr | 54 |

This result is better than the previously reported IC50 values of secondary hydrolysates obtained using a similar substrate but the Alcalase/EndoPro enzyme combination (IC50= 27 μg/ml; see WO2006/025731).

In an attempt to even further improve the ACE-inhibitory activity of a whey-derived hydrolysate, it was decided to prepare a secondary hydrolysate from a primary hydrolysate with a DH of either 3.0% or 7.0%. After prolonged incubation of the primary hydrolysates with thermolysin (Thermoase; 23 hr, E/S = 1/50) the IC50 values were determined. When the primary hydrolysate had a DH of 3%, the obtained IC50 value was approx. 23 μg/ml, i.e. was exactly the same as was found for DH=5.0%. In contrast, when the primary hydrolysate had a DH of 7%, the obtained Ic50 value was somewhat higher, approximately 27 μg/ml. Thus, for optimal results using neutrase as first enzyme, the degree of hydrolysis of the first hydrolysate appears to have a maximum value of approximate 5%.

When a common WPC (Hiprotal 875) was used containing the whey proteins BLG, ALA, CMP, IgG and BSA as whey-containing substrate in a method of the invention using the same conditions as described above, a final IC50 value of 16 µg/ml was reached.

## Example 5: Isolation and Identification of ACE-inhibitory peptides

A portion of secondary hydrolysate from Example 4 was produced and dried after final protease treatment. For the isolation of peptides, the dried hydrolysate was dissolved in phosphate-saline buffer, (50mM, pH=6.0, 0.15 M sodium chloride) at a final protein/peptide concentration of 20 mg/ml. The hydrolysate was centrifuged after dissolution in order to remove all particles from solution. 5 ml of this solution was applied on a gel filtration column (Superdex 30-PG, 16/60) and subsequently eluted with the phosphate-saline buffer (50mM, pH=6.0, 0.15 M sodium chloride) at a flow rate of 1ml/ minute. Eight 20ml fractions were collected and analyzed for their ACE-inhibiting activity. Furthermore, a peptide profile of each superdex-fraction was determined with RP-HPLC.

Table 3: ACE inhibiting activity of the Superdex-fractions; 100µl of the obtained fraction was included in the inhibition assay

| FRACTION | % ACE inhibition |
| --- | --- |
| X-1 | 0 |
| X-2 | 0 |
| X-3 | 22 |
| X-4 | 55 |
| X-5 | 70 |
| X-6 | 60 |
| X-7 | 30 |
| X-8 | 0 |

Highest ACE inhibiting activity was found in fraction X5 and in fraction X6. From RP-HPLC it appeared that fraction X-6 was almost a pure peptide (1 peak in RP-HPLC chromatogram. The identity of this peak was determined by LC-MSMS and found to contain peptides VAGTWYSL and VAGTWYS.

Fraction X-5 was further purified by RP-HPLC. The pH of fraction X-5 was decreased by the addition of formic acid. Approximately 10ml was applied on a preparative reversed phase column (Resource RPC-3ml,). After rinding the column with buffer, the adsorbed peptides were thereafter selectively eluted from the RP-column with an acetonitril gradient in phosphate-saline buffer (50mM, pH=6.0, 0.15 M sodium chloride). The eluted peptides were collected in fractions (size 1 ml). To obtain a sufficient amount of peptides for subsequent analysis, this procedure was repeated 5 times.

After drying and subsequent dissolution of the peptides in ACE-inhibitory buffer, ACE-inhibiting activity of these fractions was determined. Furthermore main peptide constituents of the fractions were identified with LC-MSMS or MALDI-TOF.

The main peptides present in fraction X-5 were found to be LDIQK, WYSL, IAEKTKIPAV, AASDISL, VAGTW, ASDISL, VYVEE, LKALPM, LIVTQT, IIVTQT, DAQSAPL, VLDTDY, LVLDTDYKKY, DTDYKKY and LKPTPEG.

**Claims**

1. A method for providing a protein hydrolysate having angiotensin-converting enzyme (ACE)-inhibitory activity, comprising:

    - treating a whey protein-containing substrate with a bacterial heat-labile neutral protease to produce a primary hydrolysate; and
    - treating said primary hydrolysate with a thermolysin to produce a secondary hydrolysate.

2. Method according to claim 1, wherein said neutral protease is derived from *Bacillus subtilis* or from *Bacillus amyloliquefaciens.*,

3.  Method according to claim 1 or 2, wherein said neutral protease the neutral protease marketed under the tradename Neutrase(R) or an enzyme with similar properties.

4.  A method according to any one of claims 1 to 3, wherein said thermolysin is a bacterial neutral heat-stable metalloproteinase (EC 3.4.24.4 or EC 3.4.24.27) derived from *B. stearothermophilus (B. thermoproteolyticus* variant Rokko) or a thermolysin with similar properties.

5.  A method according to any one of claims 1-4, comprising inactivation of bacterial heat-labile neutral protease prior to treating the primary hydrolysate with thermolysin.

6.  A method according to any one of claims 1-5, wherein the enzyme to substrate ratio (E/S) in the reaction mixture of the first and/or second hydrolysis ranges from 1/20 to 1/250, preferably 1/50 to 1/100.

7.  A method according to any one of claims 1 to 6, wherein said substrate is a whey protein concentrate (WPC), preferably a WPC which has been enriched with beta-lactoglobulin (BLG).

8.  A method according to any one of claims 1 to 7, wherein the whey protein-containing substrate contains at least 25%, preferably at least 35%, more preferably at least 60% BLG, based on the total weight of the substrate.

9.  A method according to any one of claims 1-8, wherein the whey-protein concentration of the first and/or second hydrolysate ranges from about 1 to about 50 mg per 100 ml, preferably from about 2 to about 15 mg/ml.

10. A method according to any one of claims 1-9, wherein the first reaction step is carried out until a degree of hydrolysis (DH) of about 10 has been reached, preferably about 3-7%, more preferably until DH is approximately 3-5%.

11. A method according to any one of claims 1-10, wherein the treatment with said neutral protease is carried out at around 50°C and at a pH of around 7, preferably for approximately 3-4 hours.

12. A method according to any one of claims 1-11, wherein the treatment with said thermolysin is carried out at around 50-60°C and at a pH of about 8, preferably for at least 8 hours, more preferably at least 12 hours.

13. A method according to any one of claims 1-12, further comprising the steps of fractionating the secondary lysate into individual fractions comprising one or more peptides and identifying the amino acid sequence of at least one peptide having ACE-inhibitory activity.

14. A whey hydrolysate having angiotensin-converting enzyme (ACE)-inhibitory activity, obtainable with a method according to any one of claims 1-12.

15. An ACE-inhibitory peptide obtainable by a method according to claim 13.

16. Peptide according to claim 15, selected from the group consisting of the peptides VAGTWYS, VAGTWYSL, WYSL, IAEKTKIPAV, AASDISL, VAGTW, ASDISL, VYVEE, LKALPM, LIVTQT, IIVTQT, DAQSAPL, VLDTDY, LVLDTDYKKY, DTDYKKY, LKPTPEG, and functional peptides analog thereof having ACE-inhibitory activity.

17. A composition comprising a hydrolysate according to claim 14 and/or at least one peptide according to claim 15 or 16, and a suitable carrier.

18. Composition according to claim 17, wherein said composition is a pharmaceutical composition or a food or luxury food.

19. Composition according to claim 17 or 18, wherein the composition is a liquid product, such as a milk product or a fruit juice, or a solid product, such as a powder.

20. Use of a hydrolysate according to claim 14 or a peptide according to claim 15 or 16 for the preparation of a medicament for inhibiting ACE activity in a mammal, for lowering the blood pressure, and/or for preventing the occurrence of hypertension.

Figure 1:

Figure 2:

**European Patent Office**

**EUROPEAN SEARCH REPORT**

Application Number

EP 07 10 3545

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/004876 A (CAMPINA NEDERLAND HOLDING B V [NL]; CADEE JENNEKE ADRIANA [NL]; MALLEE) 11 January 2007 (2007-01-11) <br> * page 1, line 2 - line 3 * <br> * page 4 * <br> * page 5 * <br> * page 6, line 29 - line 33 * <br> * page 7, line 34 - line 39 * <br> * page 9, line 18 - line 20 * <br> ----- | 1-14, 17-20 | INV. <br> C07K5/117 <br> C07K7/00 <br> C12P21/06 <br> A23L1/305 <br> A23J3/34 <br> A61K38/01 <br> A61K38/02 <br> A61K38/04 |
| D,Y | WO 02/19837 A (NEW ZEALAND DAIRY BOARD [NZ]; SCHLOTHAUER RALF CHRISTIAN [NZ]; SCHOLLU) 14 March 2002 (2002-03-14) <br> * page 4 * <br> * claims 1-14,17-20 * <br> ----- | 1-14, 17-20 | |
| Y | WO 99/65326 A (NEW ZEALAND DAIRY BOARD [NZ]; SCHLOTHAUER RALF CHRISTIAN [NZ]; SCHOLLU) 23 December 1999 (1999-12-23) <br> * claims 1-13 * <br> * page 8, line 5 - line 15 * <br> ----- | 1-14, 17-20 | TECHNICAL FIELDS SEARCHED (IPC) |
| D,Y | WO 2006/025731 A (FRIESLAND BRANDS BV [NL]; DE SLEGTE JAAP [NL]; KLARENBEEK GIJSBERTUS []) 9 March 2006 (2006-03-09) <br> * page 4; claims 1-16 * <br> ----- | 1-14, 17-20 | C07K <br> C12P <br> A23L <br> A23J <br> A61K |
| Y | WO 2004/057976 A (CANADA NATURAL RESOURCES [CA]; WU JIANPING [CA]; MUIR ALISTER D [CA];) 15 July 2004 (2004-07-15) <br> * page 14, line 17 - line 30 * <br> * page 15, line 9 - line 19 * <br> * page 19; example 9 * <br> * claims 1-45 * <br> ----- | 1-14, 17-20 | |
| Y | US 6 787 168 B1 (SAWHILL JAMES W [US]) 7 September 2004 (2004-09-07) <br> * the whole document * <br> ----- | 1-14, 17-20 | |

-/--

~~The present search report has been drawn up for all claims~~

| Place of search | Date of completion of the search | Examiner |
|---|---|---|
| Berlin | 20 September 2007 | Hagenmaier, Susanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**EUROPEAN SEARCH REPORT**

Application Number

EP 07 10 3545

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ABUBAKAR AMHAR ET AL: "New derivation of the inhibitory activity against angiotensin converting enzyme (ACE) from sweet cheese whey" TOHOKU JOURNAL OF AGRICULTURAL RESEARCH, vol. 47, no. 1-2, 1996, pages 1-8, XP008083421 ISSN: 0040-8719 * the whole document * | 1-14, 17-20 | |
| A | HERNÁNDEZ-LEDESMA B ET AL: "Angiotensin converting enzyme inhibitory activity in commercial fermented products. Formation of peptides under simulated gastrointestinal digestion" JOURNAL OF AGRICULTURAL AND FOOD CHEMISTRY, AMERICAN CHEMICAL SOCIETY. WASHINGTON, US, vol. 52, no. 6, 24 March 2004 (2004-03-24), pages 1504-1510, XP008057959 ISSN: 0021-8561 * the whole document * | | |
| | | | TECHNICAL FIELDS SEARCHED (IPC) |
| D,A | MEISEL H ET AL: "Bioactive peptides encrypted in milk proteins: proteolytic activation and thropho-functional properties." ANTONIE VAN LEEUWENHOEK 1999 JUL-NOV, vol. 76, no. 1-4, July 1999 (1999-07), pages 207-215, XP002960560 ISSN: 0003-6072 * the whole document * | | |

~~The present search report has been drawn up for all claims~~

| Place of search | Date of completion of the search | Examiner |
|---|---|---|
| Berlin | 20 September 2007 | Hagenmaier, Susanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent

Office

Application Number

EP 07 10 3545

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14 (all completely), 17-20 (all partially)

☐ The present supplementary European search report has been drawn up for those parts of the European patent application which relate to the invention first mentioned in the claims (Rule 164 (1) EPC).

| | | |
|---|---|---|
| **European Patent** **Office** | **LACK OF UNITY OF INVENTION** **SHEET B** | Application Number EP 07 10 3545 |

The Search Division considers that the present European patent application does not comply with the requirements of unity of invention and relates to several inventions or groups of inventions, namely:

1. claims: 1-14 (all completely), 17-20 (all partially)

   A method of providing a protein hydrosylate having angiotensin-converting enzyme (ACE)-inhibitory activity, comprising treating a whey protein-containing substrate with a bacterial heat-labile neutral protease to produce a primary hydrosylate; and treating said primary hydrosylate with a thermolysin to produce a secondary hydrosylate; a whey hydrosylate having ACE-inhibitory activity, obtainable by said method, composition comprising said hydrosylate and use of said hydrosylate for the preparation of a medicament for inhibiting ACE activity in a mammal.
   ---

2. claims: 15-20 (all partially)

   An ACE-inhibitory peptide selected from the group VAGTWYS, VAGTWYSL, WYSL and VAGTW, composition comprising said peptide and use of said peptide for the preparation of a medicament for inhibiting ACE activity in a mammal.
   ---

3. claims: 15-20 (all partially)

   The ACE-inhibitory peptide IAEKTKIPAV , a composition comprising said peptide and use of said peptide for the preparation of a medicament for inhibiting ACE activity in a mammal.
   ---

4. claims: 15-20 (all partially)

   An ACE-inhibitory peptide selected from the group AASDISL and  ASDISL, a composition comprising said peptide and use of said peptide for the preparation of a medicament for inhibiting ACE activity in a mammal.
   ---

5. claims: 15-20 (all partially)

   The ACE-inhibitory peptide VYVEE, a composition comprising said peptide and use of said peptide for the preparation of a medicament for inhibiting ACE activity in a mammal.
   ---

6. claims: 15-20 (all partially)

   The ACE-inhibitory peptide LKALPM , a composition comprising said peptide and use of said peptide for the preparation of a medicament for inhibiting ACE activity in a mammal.
   ---

**European Patent Office**

**LACK OF UNITY OF INVENTION SHEET B**

Application Number

EP 07 10 3545

The Search Division considers that the present European patent application does not comply with the requirements of unity of invention and relates to several inventions or groups of inventions, namely:

7. claims: 15-20 (all partially)

    An ACE-inhibitory peptide selected from the group LIVTQT and IIVTQT, a composition comprising said peptide and use of said peptide for the preparation of a medicament for inhibiting ACE activity in a mammal.

      ---

8. claims: 15-20 (all partially)

    The ACE-inhibitory peptide DAQSAPL, a composition comprising said peptide and use of said peptide for the preparation of a medicament for inhibiting ACE activity in a mammal.

      ---

9. claims: 15-20 (all partially)

    An ACE-inhibitory peptide selected from the group VLDTDY, LVLDTDYKKY and DTDYKKY, a composition comprising said peptide and use of said peptide for the preparation of a medicament for inhibiting ACE activity in a mammal.

      ---

10. claims: 15-20 (all partially)

    The ACE-inhibitory peptide LKPTPEG, a composition comprising said peptide and use of said peptide for the preparation of a medicament for inhibiting ACE activity in a mammal.

      ---

## ANNEX TO THE EUROPEAN SEARCH REPORT
## ON EUROPEAN PATENT APPLICATION NO.

EP 07 10 3545

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2007

| Patent document cited in search report | | | Publication date | Patent family member(s) | | | Publication date |
|---|---|---|---|---|---|---|---|
| WO 2007004876 | A | | 11-01-2007 | NONE | | | |
| WO 0219837 | A | | 14-03-2002 | AU | 9037601 | A | 22-03-2002 |
| | | | | BR | 0113812 | A | 01-02-2005 |
| | | | | CA | 2421714 | A1 | 14-03-2002 |
| | | | | CN | 1474656 | A | 11-02-2004 |
| | | | | EP | 1317186 | A1 | 11-06-2003 |
| | | | | HK | 1063132 | A1 | 21-04-2006 |
| | | | | HU | 0302370 | A2 | 28-10-2003 |
| | | | | JP | 2004508025 | T | 18-03-2004 |
| | | | | MX | PA03002109 | A | 24-05-2004 |
| | | | | NO | 20031096 | A | 07-05-2003 |
| | | | | NZ | 506866 | A | 30-05-2003 |
| | | | | ZA | 200301982 | A | 25-06-2004 |
| WO 9965326 | A | | 23-12-1999 | AT | 275831 | T | 15-10-2004 |
| | | | | AU | 761477 | B2 | 05-06-2003 |
| | | | | AU | 4535999 | A | 05-01-2000 |
| | | | | DE | 69920219 | D1 | 21-10-2004 |
| | | | | DE | 69920219 | T2 | 22-09-2005 |
| | | | | DK | 1087668 | T3 | 24-01-2005 |
| | | | | EP | 1087668 | A1 | 04-04-2001 |
| | | | | NO | 20006305 | A | 14-02-2001 |
| | | | | NZ | 508867 | A | 28-11-2003 |
| | | | | TW | 225490 | B | 21-12-2004 |
| | | | | US | 2004086958 | A1 | 06-05-2004 |
| | | | | US | 6919314 | B1 | 19-07-2005 |
| WO 2006025731 | A | | 09-03-2006 | EP | 1794189 | A1 | 13-06-2007 |
| | | | | NL | 1026931 | C2 | 01-03-2006 |
| WO 2004057976 | A | | 15-07-2004 | AU | 2003289794 | A1 | 22-07-2004 |
| | | | | BR | 0317762 | A | 22-11-2005 |
| | | | | CA | 2508219 | A1 | 15-07-2004 |
| | | | | CN | 1731934 | A | 08-02-2006 |
| | | | | EP | 1575375 | A1 | 21-09-2005 |
| | | | | JP | 2006512371 | T | 13-04-2006 |
| | | | | MX | PA05006403 | A | 05-12-2005 |
| US 6787168 | B1 | | 07-09-2004 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03102195 A
- WO 0219837 A
- JP 06128287 B
- US 6998259 B
- WO 2006025731 A
- WO 2006005757 A
- WO 04098310 A

**Non-patent literature cited in the description**

- **NAKAMURA, Y. et al.** *J. Dairy Sci.,* 1996, vol. 78, 777-783
- **MEISEL ; BOCKELMANN.** *Ant. van Leeuwen.,* 1999, vol. 76, 207-215
- **GOBBETTI et al.** *Appl. Envir. Microbiol.,* 2000, vol. 66, 3898-3904
- The peptides, Analysis, Synthesis, Biology. Academic Press, 1981, vol. 3
- *Pure and Applied Chemistry,* 1987, vol. 59 (3), 331-344
- The Peptides, Analysis, Synthesis, Biology. Acad. Press, 1980, vol. 2
- **KNEIB-CORDONIER ; MULLEN.** *Int. J. Peptide Protein Res.,* 1987, vol. 30, 705-739
- **FIELDS ; NOBLE.** *Int. J. Peptide Protein Res.,* 1990, vol. 35, 161-214
- **H.D. JAKUBKE.** The Peptides, Analysis, Synthesis, Biology. Acad. Press, 1987, vol. 9

Annex to the application documents - subsequently filed sequences listing

SEQUENCE LISTING

<110> Friesland Brands B.V.

<120> Methods for producing ACE-inhibitory peptides from whey and peptides obtained thereby

<130> P78465EP00

<140> EP 07103545.5
<141> 2007-03-06

<160> 17

<170> PatentIn version 3.3

<210> 1
<211> 7
<212> PRT
<213> Artificial

<220>
<223> ACE-inhibitory peptide 1

<400> 1

Val Ala Gly Thr Trp Tyr Ser
1               5


<210> 2
<211> 8
<212> PRT
<213> Artificial

<220>
<223> ACE-inhibitory peptide 2

<400> 2

Val Ala Gly Thr Trp Tyr Ser Leu
1               5


<210> 3
<211> 5
<212> PRT
<213> Artificial

<220>
<223> ACE-inhibitory peptide 3

<400> 3

Leu Asp Ile Gln Lys
1               5


<210> 4
<211> 4
<212> PRT
<213> Artificial

<220>
<223> ACE-inhibitory peptide 4

<400> 4

```
Trp Tyr Ser Leu
1


<210>   5
<211>   10
<212>   PRT
<213>   Artificial

<220>
<223>   ACE-inhibitory peptide 5

<400>   5

Ile Ala Glu Lys Thr Lys Ile Pro Ala Val
1               5               10


<210>   6
<211>   7
<212>   PRT
<213>   Artificial

<220>
<223>   ACE-inhibitory peptide 6

<400>   6

Ala Ala Ser Asp Ile Ser Leu
1               5


<210>   7
<211>   5
<212>   PRT
<213>   Artificial

<220>
<223>   ACE-inhibitory peptide 7

<400>   7

Val Ala Gly Thr Trp
1               5


<210>   8
<211>   6
<212>   PRT
<213>   Artificial

<220>
<223>   ACE-inhibitory peptide 8

<400>   8

Ala Ser Asp Ile Ser Leu
1               5


<210>   9
<211>   5
<212>   PRT
<213>   Artificial

<220>
<223>   ACE-inhibitory peptide 9
```

```
<400>  9

Val Tyr Val Glu Glu
1              5


<210>  10
<211>  6
<212>  PRT
<213>  Artificial

<220>
<223>  ACE-inhibitory peptide 10

<400>  10

Leu Lys Ala Leu Pro Met
1              5


<210>  11
<211>  6
<212>  PRT
<213>  Artificial

<220>
<223>  ACE-inhibitory peptide 11

<400>  11

Leu Ile Val Thr Gln Thr
1              5


<210>  12
<211>  6
<212>  PRT
<213>  Artificial

<220>
<223>  ACE-inhibitory peptide 12

<400>  12

Ile Ile Val Thr Gln Thr
1              5


<210>  13
<211>  7
<212>  PRT
<213>  Artificial

<220>
<223>  ACE-inhibitory peptide 13

<400>  13

Asp Ala Gln Ser Ala Pro Leu
1              5


<210>  14
<211>  6
<212>  PRT
<213>  Artificial

<220>
```

```
<223>   ACE-inhibitory peptide 14

<400>   14

Val Leu Asp Thr Asp Tyr
1                   5


<210>   15
<211>   10
<212>   PRT
<213>   Artificial

<220>
<223>   ACE-inhibitory peptide 15

<400>   15

Leu Val Leu Asp Thr Asp Tyr Lys Lys Tyr
1               5                   10


<210>   16
<211>   7
<212>   PRT
<213>   Artificial

<220>
<223>   ACE-inhibitory peptide 16

<400>   16

Asp Thr Asp Tyr Lys Lys Tyr
1               5


<210>   17
<211>   7
<212>   PRT
<213>   Artificial

<220>
<223>   ACE-inhibitory peptide 17

<400>   17

Leu Lys Pro Thr Pro Glu Gly
1               5
```